# EUROPEAN PATENT SPECIFICATION

(11) **EP 3 722 790 B1**
(45) Date of publication and mention of the grant of the patent: **09.08.2023**
(21) Application number: 18899600.3
(22) Date of filing: 13.11.2018
(51) Int. Cl.: G01N 21/78, A61B 5/157, G01N 33/52

(54) **COMPONENT MEASUREMENT SYSTEM, MEASUREMENT DEVICE, AND MEASUREMENT TIP**
KOMPONENTENMESSSYSTEM, MESSVORRICHTUNG UND MESSSPITZE
SYSTÈME DE MESURE DE COMPOSANT, DISPOSITIF DE MESURE ET POINTE DE MESURE

(30) Priority: 15.01.2018 JP 2018004358
(43) Date of publication of application: 14.10.2020
(73) Proprietor: Terumo Kabushiki Kaisha, Tokyo 151-0072 (JP)
(72) Inventor: MORIUCHI, Takeyuki, Nakakoma-gun, Yamanashi 409-3853 (JP); AIKAWA, Ryokei, Ashigarakami-gun, Kanagawa 259-0151 (JP)
(74) Representative: Casalonga
(86) International application number: PCT/JP2018/041923
(87) International publication number: WO 2019/138681

(56) References cited:
- EP-A1- 3 124 957
- WO-A1-99/45385
- WO-A1-2016/147527
- WO-A1-2016/152225
- JP-A- 2003 518 607
- JP-A- 2007 010 500
- JP-A- 2012 500 968
- JP-A- 2015 533 211
- JP-A- 2017 508 158
- US-A- 5 504 011

## Description

### Technical Field

The present invention relates to a component measurement system a measurement device, and a measurement tip, which measure a component contained in a liquid. In particular, the present invention relates to a component measurement system according to the preambles of independent claims 1 and 2, such as it is for example known from WO99/45385 A1.

### Background Art

In the related art, in order to diagnose diabetes and determine a dose of insulin, a component measurement system (blood glucose meter) for measuring a glucose component contained in blood has been used. For example, JP-A-2000-171427 discloses a blood glucose meter as follows. A measurement tip is mounted on a measurement device, and the blood fetched into the measurement tip is caused to react with a reagent, and a colored degree thereof is detected by a detection unit of an optical system. In this way, the blood glucose meter adopts a so-called colorimetric detection principle.

### Summary of Invention

Incidentally, according to a colorimetric component measurement system, the following fact is known. During a process of calculating a component from a detection result acquired by a detection unit, the component measurement system is greatly affected by a length of light passing through a medium (in other words, a clearance (spatial clearance) of a measurement target region where a liquid flows inside a measurement tip). Therefore, if the spatial clearance of the measurement tip is changed due to a manufacturing error, an accurate detection result cannot be obtained. However, the measurement tips are produced on a large scale, and it is not realistic to completely eliminate the manufacturing error. Accordingly, it is necessary to provide more special means to perform more accurate measurement.

The component measurement system disclosed in JP-A-2000-171427 is configured as follows. A cutout portion (information holding means) including information such as a correction value and a lot is disposed in the measurement tip, and the measurement device reads the information of the cutout portion to correct a measurement value. However, if the cutout portion is retrofitted in manufacturing the measurement tip, another error (such as a change in the clearance) may occur in the measurement tip. Moreover, the cutout portion may lead to cost increasing when the measurement tips are produced on a large scale.

That is, according to the colorimetric component measurement system, the measurement tip needs to easily hold information relating to the clearance (spatial clearance), and needs to transmit the information to the measurement device so that the information is used in calculating the component.

The present invention is made in view of the above-described circumstances, and an object thereof is to provide component measurement systems which are capable of easily providing or acquiring information relating to a spatial clearance so that a component of a liquid can be more accurately measured.

According to the present invention, in order to achieve the above-described object, there are provided component measurement systems according to independent claims 1 and 2. The dependent claims relate to advantageous embodiments.

According to the present invention, the measurement tip can easily provide the information relating to the spatial clearance by using the printing layer. On the other hand, the measurement device can easily acquire the information relating to the spatial clearance by using the reading unit. In this manner, the component measurement system can accurately measure the component of the liquid, based on each spatial clearance of the measurement tip. As a result, for example, a health care worker can more satisfactorily perform treatment corresponding to a measurement result of a body fluid component of a patient. In particular, the printing layer can be easily provided at low cost without causing a manufacturing error after the tip body is manufactured, thereby contributing to mass production of the measurement tip.

### Brief Description of Drawings

[Fig. 1] Fig. 1 is a plan view schematically illustrating an overall configuration of a component measurement system (blood glucose meter) according to a first embodiment of the present invention.
[Fig. 2] Fig. 2 is a perspective view for describing a tip body of a measurement tip.
[Fig. 3] Fig. 3A is a plan view of the measurement tip. Fig. 3B is a sectional view taken along line IIIB-IIIB in Fig. 3A. Fig. 3C is a sectional view taken along line IIIC-IIIC in Fig. 3A.
[Fig. 4] Fig. 4 is a view for schematically describing an internal configuration of a measurement device.
[Fig. 5] Fig. 5 is a block diagram illustrating a functional unit of a control unit.
[Fig. 6] Fig. 6 is a view for describing an example of an associated map.
[Fig. 7] Fig. 7A is a flowchart illustrating a manufacturing flow of the measurement tip. Fig. 7B is a flowchart illustrating a flow of measuring a blood glucose level in the component measurement system.
[Fig. 8] Fig. 8A is a side sectional view illustrating a state of the measurement device before the measurement tip is inserted. Fig. 8B is a side sectional view illustrating a state where the measurement tip is inserted into the measurement device.
[Fig. 9] Fig. 9 is a graph illustrating a change in an absorbance detected by the control unit when the measurement tip is inserted.
[Fig. 10] Fig. 10A is a side sectional view illustrating a state where the measurement tip is completely mounted. Fig. 10B is a side sectional view illustrating a measurement operation when blood is collected by the measurement tip.
[Fig. 11] Fig. 11A is a plan view illustrating a measurement tip of a component measurement system (blood glucose meter) according to a second embodiment of the present invention. Fig. 11B is an enlarged plan view illustrating an example of a printing layer of the measurement tip, and illustrates an example of an absorbance corresponding to the printing layer.
[Fig. 12] Fig. 12A is a side sectional view of a component measurement system (blood glucose meter) according to a third embodiment of the present invention. Fig. 12B is a plan view illustrating a measurement tip.
[Fig. 13] Fig. 13A is a side sectional view of a component measurement system (blood glucose meter) according to a fourth embodiment of the present invention. Fig. 13B is a plan view illustrating a measurement tip.
[Fig. 14] Fig. 14 is a plan view schematically illustrating an overall configuration of a component measurement system (blood glucose meter) according to a non-claimed example.

### Description of Embodiments

Hereinafter, preferred embodiments according to the present invention will be described in detail with reference to the accompanying drawings.

### [First Embodiment]

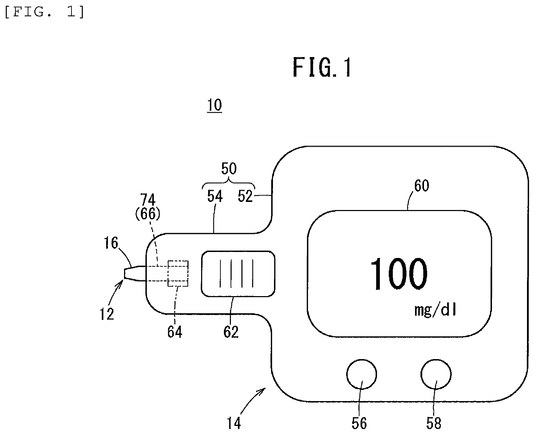

As illustrated in Fig. 1, a component measurement system 10 according to a first embodiment of the present invention is configured to serve as a blood glucose meter that measures the amount of glucose component in blood (blood glucose level). Therefore, hereinafter, the component measurement system 10 is also referred to as a blood glucose meter 10.

The blood glucose meter 10 includes a measurement tip 12 capable of collecting blood of a patient, and a measurement device 14 on which the measurement tip 12 is mounted, and which measures the blood collected by the measurement tip 12. The measurement tip 12 is configured to serve as a disposable type that is discarded after each measurement. The measurement device 14 is configured to serve as a reuse type capable of repeatedly measuring the blood glucose level by replacing the measurement tip 12.

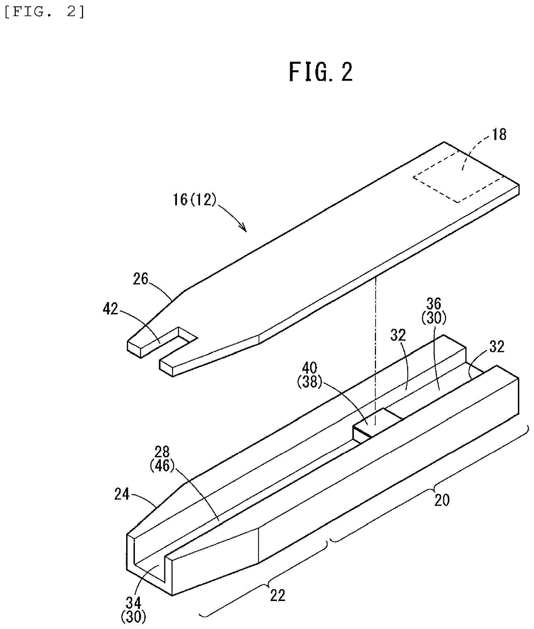

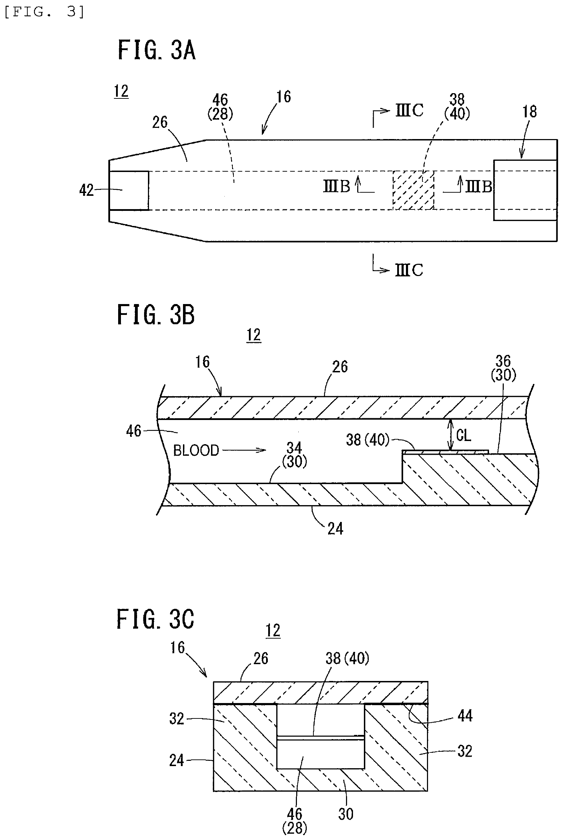

As illustrated in Figs. 2 and 3A to 3C, the measurement tip 12 of the blood glucose meter 10 has a tip body 16 having an elongated plate shape as a whole and to be inserted into the measurement device 14, and a printing layer 18 printed at a predetermined position (surface of a proximal portion in the present embodiment) of the tip body 16. In a mounted state of the measurement device 14, a portion (insertion range 20) of the tip body 16 is disposed inside the measurement device 14, and the other portion (exposure range 22) is in a state of protruding outward of the measurement device 14. In this manner, attachment and detachment operations are easily performed with a user's hand. According to a configuration in which an eject lever 62 is installed in the measurement device 14, the measurement tip 12 may be mounted without protruding outward of the measurement device 14. The exposure range 22 has a trapezoidal shape in which a rightward-leftward width is slightly narrowed toward a distal direction. The tip body 16 fetches the blood into the measurement tip 12 from a distal portion of the exposure range 22. In this manner, the blood is caused to flow in a proximal direction (insertion range 20 side), and the blood glucose level can be measured inside the measurement device 14.

More specifically, the tip body 16 is configured so that two resin members (a lower member 24 and an upper member 26) are stacked on each other. The lower member 24 and the upper member 26 are formed in the mutually same shape in a plan view (rectangular shape in which both corners of a distal end of the exposure range 22 are cut out).

As a material for forming the lower member 24 and the upper member 26, it is preferable to adopt a thermoplastic resin material which can achieve high molding accuracy through heating. For example, the thermoplastic resin material includes polymer materials such as polyethylene (including those which are formed of cyclic polyolefin), polypropylene, polyethylene terephthalate, polyvinyl chloride, polystyrene, ABS resin, acrylic, polyamide, polyester, and fluororesin, or a mixture thereof. In particular, adopting the acrylic (including PMMA) is an effective way in view of high transparency and excellent impact resistance. As a matter of course, for example, various materials such as glass and quartz may be adopted in addition to the thermoplastic resin material.

As a whole, the lower member 24 is formed to be thicker than or equal to the upper member 26, and a central portion in a width direction thereof has a groove portion 28 formed along a longitudinal direction. Specifically, in the lower member 24, the groove portion 28 is configured to include a bottom wall 30 extending in the longitudinal direction, and a pair of side walls 32 protruding in an upward direction (direction toward the upper member 26) from both sides in the width direction of the bottom wall 30 and extending in the longitudinal direction.

Then, the bottom wall 30 has a first step portion 34 extending to have a constant thickness from a distal portion to an intermediate position in the proximal direction, and a second step portion 36 extending to be thicker than the first step portion 34 from the intermediate position to the proximal end. That is, the bottom wall 30 is formed in a step shape. A cross-sectional area of the groove portion 28 in the first step portion 34 is larger than a cross-sectional area of the groove portion 28 in the second step portion 36.

The groove portion 28 configured to include a distal side of the second step portion 36 is set to a detection target region 38 to be irradiated with the measurement light of the measurement device 14. A reagent 40 (bloodreactive agent) for coloring the blood is applied to the distal side (detection target region 38) of the second step portion 36. The tip body 16 has this shape. Accordingly, even in a case where a body fluid as a specimen has high viscosity (for example, whole blood of high hematocrit), the body fluid having the high viscosity can quickly flow to the detection target region 38 to which the reagent 40 is applied. Therefore, an advantageous effect is achieved in that a specimen behavior is uniformed inside the tip body 16. At the same time, the amount of information of the measurement light transmitted through a spatial clearance CL in the detection target region 38 can be increased, and an advantageous effect is achieved in that the component of the specimen can be more accurately obtained. Furthermore, the application amount of the reagent 40 can be reduced, and a cost reduction effect is obtained. Accordingly, it is possible to provide the highly accurate measurement tip 12 at low cost.

For example, the reagent 40 is disposed to have an entire width and a predetermined longitudinal length of the groove portion 28. A proximal side of an application position of the reagent 40 has no particular one, and the second step portion 36 extends as it is. The reagent 40 is not particularly limited as long as the reagent 40 is colored by reacting with the component of the liquid. In the present embodiment (configuration for measuring the blood glucose level), for example, the reagent 40 includes an enzyme reagent such as glucose oxidase (GOD) and peroxidase (POD), or a color-forming reagent such as 4-aminoantipyrine and N-ethyl N-(2-hydroxy-3-sulfopropyl)-m-toluidine. Alternatively, the reagent 40 may be appropriately selected depending on properties or measurement wavelengths of a measurement target component.

On the other hand, the upper member 26 is formed in a long plate extending to have a constant plate thickness, and a distal portion thereof has a cutout 42 formed in a rectangular shape.

The tip body 16 is configured as follows. The upper member 26 is stacked on the lower member 24, and both of these are fixed to each other by using proper fixing means (adhesive 44 in the present embodiment) such as an adhesive, a tape, ultrasound fusion, and heat-welding. In order to adjust and equalize a clearance (distance from the bottom wall 30 to the upper member 26) in the first step portion 34 and the second step portion 36, both of these may be fixed to each other after a spacer having any desired thickness is disposed therein. A double-sided tape that also functions as the adhesive and the spacer may also be used. In a stacked state, the groove portion 28 of the lower member 24 is covered by the upper member 26, thereby forming a blood flow path 46 (detection space) for fetching the blood. The blood flow path 46 communicates with the cutout 42 formed in the upper member 26. That is, the cutout 42 functions as an opening that initially fetches the blood of the patient.

In addition, as described above, an interval between the bottom wall 30 and the upper member 26 is a spatial clearance of the blood flow path 46 through which the blood flows. The spatial clearance of the blood flow path 46 is large in the first step portion 34, and is small in the second step portion 36. The clearance in the first step portion 34 is not particularly limited. However, since even a high-viscosity specimen can properly flow therethrough, it is preferable that the clearance is 50 µm to 200 µm. Since a specimen inflow speed is fast, it is particularly preferable that the clearance is approximately 60 µm to 100 µm. The clearance in the second step portion 36 is appropriately set depending on the purpose. However, it is preferable that the clearance is 30 µm to 80 µm in a case of component measurement. That is, the clearance of the first step portion 34 is preferably 1.3 to 5.0 times the clearance of the second step portion 36. If the ratio is 1.3 to 2.5 times, a difference is reduced between a collected specimen and a measured specimen. Accordingly, it is particularly preferable, since both the specimen inflow speed and the measurement accuracy can be compatibly achieved. These intervals are determined, based on a size of the tip body 16, the blood used for the measurement, and a design of a photometric block 54 (to be described later). As the interval is shorter, a blood required amount is smaller. Accordingly, a burden on the patient such as pain is reduced, and at the same time, the smaller interval contributes to a downsized device. However, the amount of information used for the measurement is reduced. Consequently, it is difficult to improve the measurement accuracy. For example, in the present embodiment, the clearance of the first step portion 34 is set to 80 µm, the clearance of the second step portion 36 is set to 40 µm, and the clearance of the first step portion 34 is set to twice the clearance of the second step portion 36.

As described above, the reagent 40 is applied to a distal position of the second step portion 36. The interval of the blood flow path 46 (detection space) from an upper surface of the reagent 40 to the upper member 26 (hereinafter, referred to as the spatial clearance CL) is an interval along a traveling direction of measurement light ml (to be described later), and is a parameter when the measurement device 14 calculates the blood glucose level. For example, it is preferable that the spatial clearance CL is 20 µm to 50 µm. In the present embodiment, the spatial clearance CL is designed to be basically 40 µm. The printing layer 18 (to be described later) may have information on the spatial clearance CL' including the thickness of the lower member 24, the upper member 26, and the reagent 40, and may have the information on the spatial clearance CL obtained by separately calculating the thickness of the lower member 24, the upper member 26, and the reagent 40, then excluding the thickness from the spatial clearance CL' including the thickness of the members, and accurately calculating the length of the light passing through a medium.

Here, there is a possibility that the spatial clearance CL may not be constant due to a manufacturing error of the tip body 16 (for example, a molding error of the upper member 26 and the lower member 24, a change in the thickness of the adhesive 44, or a change in the thickness of the applied reagent 40). Therefore, in the measurement tip 12 according to the present embodiment, the spatial clearance CL is measured after manufacturing, and the printing layer 18 which holds information relating to the spatial clearance CL is provided.

The printing layer 18 is formed on one flat surface of the tip body 16 (upper member 26 in Fig. 2). In addition, the printing layer 18 may be formed to have a predetermined length in the distal direction from the proximal end of the measurement tip 12, and a width larger than a width of the blood flow path 46 formed inside the measurement tip 12. After the spatial clearance CL is measured, the printing layer 18 is printed on the upper member 26 in a state (color, transmittance (density), or pattern) corresponding to a measurement result. A configuration of the printing layer 18 will be described in detail later.

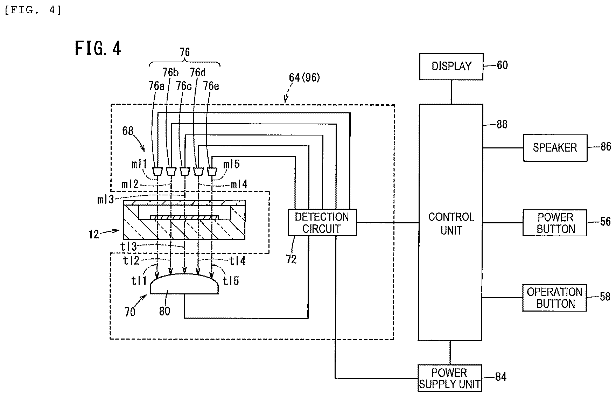

Referring back to Fig. 1, the measurement device 14 of the blood glucose meter 10 includes a housing 50 to which the measurement tip 12 is attachable and detachable, and which configures an exterior of the whole device. The housing 50 has a size that allows a user to easily perform a gripping operation, and internally has a box body portion 52 accommodating a control unit 88 (control board: refer to Fig. 4) of the blood glucose meter 10, and the photometric block 54 protruding in the distal direction from one side (distal side) of the box body portion 52. In addition, a power button 56, an operation button 58, and a display 60 are disposed on an upper surface of the box body portion 52, and the eject lever 62 is disposed on an upper surface of the photometric block 54.

The power button 56 switches between activation and deactivation of the blood glucose meter 10 under an operation of the user. In an activated state, the operation button 58 functions as an operation unit that measures the blood glucose level, displays a measurement result (including the blood glucose level in the past), and switches the display, based on the operation of the user. The display 60 is configured to include a liquid crystal or an organic EL, and displays information (blood glucose level, measurement procedure, and error display) provided for the user in the measurement of the blood glucose level. The display 60 may be configured to serve as a touch panel without providing the power button 56 and the operation button 58, or may be configured so that the information is transmitted to or received from other devices through communication means without installing the display 60, the power button 56, and the operation button 58.

The photometric block 54 of the measurement device 14 is formed in an elongated cylindrical shape so as to press the distal end against a finger of the user. The photometric block 54 internally has a detection unit 64 that optically detects the glucose component contained in the blood.

The eject lever 62 disposed in the photometric block 54 is configured to be movable in the distal direction and to automatically return in the proximal direction, and is connected to an eject pin (not illustrated) inside the photometric block 54. The eject lever 62 is operated to move in the distal direction by the user. In this manner, the eject pin is operated to push out the measurement tip 12, and the measurement tip 12 is detached from the photometric block 54.

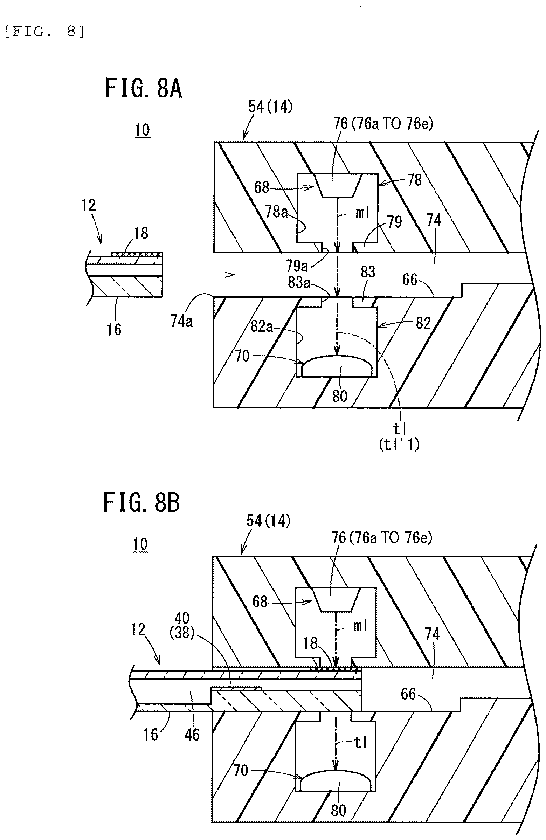

As illustrated in Fig. 4, the detection unit 64 of the measurement device 14 has a function to optically detect the component contained in the liquid. The detection unit 64 has a tip fixing portion 66 that accommodates and fixes the measurement tip 12 (refer to Fig. 8A), a light emitting unit 68 that irradiates the measurement tip 12 with the measurement light ml, a light receiving unit 70 that faces the light emitting unit 68 across the tip fixing portion 66, and a detection circuit 72 that controls the driving of the light emitting unit 68 and the light receiving unit 70. That is, the detection unit 64 is configured to serve as a transmission type optical system that emits the measurement light ml from the light emitting unit 68 and receives transmitted light tl transmitted through the measurement tip 12.

As illustrated in Fig. 8A, the tip fixing portion 66 has a mounting space 74 configured by the photometric block 54 so as to accommodate the above-described measurement tip 12. The mounting space 74 communicates with a distal opening 74a formed in the distal end of the photometric block 54, and the measurement tip 12 is inserted into the mounting space 74 through the distal opening 74a. In addition, the mounting space 74 is formed in a cross-sectional shape having the width and the thickness approximately the same as the width and the thickness of the measurement tip 12. In contrast, a depth from the distal opening 74a is formed to be shorter than the longitudinal length of the measurement tip 12. A light guide path 78a of the light emitting unit 68 and the light receiving unit 70 communicates with the mounting space 74.

As will be described later, the blood glucose meter 10 causes the detection unit 64 to perform the detection on the detection target region 38 of the measurement tip 12, and also causes the detection unit 64 to perform the detection on the printing layer 18. That is, the detection unit 64 also functions as a reading unit 96 of the printing layer 18. Therefore, the tip fixing portion 66 may have the following structure. When the measurement tip 12 is inserted, the movement of the measurement tip 12 may be temporarily stopped at a position where the printing layer 18 faces the light emitting unit 68 and the light receiving unit 70. After the movement is temporarily stopped, the measurement tip 12 may be further inserted. For example, this type structure can be realized by disposing a step that is narrowed inward on an inner surface of the photometric block 54 configuring the mounting space 74.

As illustrated in Figs. 4 and 8A, the light emitting unit 68 is disposed at a position apart as much as a predetermined distance from the distal end of the photometric block 54 (position facing the detection target region 38 in a mounted state of the measurement tip 12). The light emitting unit 68 has a plurality of (five in the present embodiment) light emitting elements 76, and a light guide portion 78 that is configured by the photometric block 54, and that guides the measurement light ml emitted from each of the light emitting elements 76 to the detection target region 38 of the measurement tip 12.

For example, a structure may be adopted as follows. Three of the light emitting elements 76 (first to fifth light emitting elements 76a to 76e) are disposed along the longitudinal direction of the photometric block 54 in a plan view. The other two of the light emitting elements 76 are disposed adjacent to each other on both sides in the width direction orthogonal to the longitudinal direction, in a state where the light emitting element 76 is a base point at the center in the longitudinal direction. That is, the first to fifth light emitting elements 76a to 76e have a cross shape as a whole. As illustrated in Fig. 4, the first to fifth light emitting elements 76a to 76e may be aligned in one row.

The first to fifth light emitting elements 76a to 76e are configured to emit the measurement light ml having peak wavelengths different from each other in a range of 495 nm to 900 nm. As an element that emits the measurement light ml having this type, an LED, an organic EL, and a laser diode may be used.

As an example, the first light emitting element 76a is configured to emit first measurement light ml1 having a peak wavelength of 650 nm. The second light emitting element 76b is configured to emit second measurement light ml2 having a peak wavelength of 810 nm. The third light emitting element 76c is configured to emit third measurement light ml3 having a peak wavelength of 750 nm. The fourth light emitting element 76d is configured to emit fourth measurement light ml4 having a peak wavelength of 540 nm. The fifth light emitting element 76e is configured to emit fifth measurement light ml5 having a peak wavelength of 560 nm.

As described above, the measurement device 14 can very accurately measure the blood glucose level by setting the first to fifth measurement light ml1 to ml5 having five different peak wavelengths. That is, a colored degree of the glucose contained in the blood is detected using the first measurement light ml1, thereby calculating a provisional blood glucose level. Influence of other components (for example, hematocrit values) is detected using the second to fifth measurement light ml2 to ml5, thereby correcting the provisional blood glucose level in a calculation process. In this manner, the very accurate blood glucose level is finally obtained. The number of the light emitting elements 76 installed in the light emitting unit 68 is not particularly limited, and one or more elements may emit the measurement light ml having a predetermined wavelength.

As illustrated in Fig. 8A, the light guide portion 78 of the light emitting unit 68 configures the light guide path 78a that guides the first to fifth measurement light ml1 to ml5 to the measurement tip 12 while reducing noise (stray light) by using the photometric block 54. The light guide portion 78 has a partition wall 79 between the mounting space 74 and the light guide path 78a. The partition wall 79 has an aperture 79a for squeezing the measurement light ml so that the first to fifth measurement light ml1 to ml5 are directed toward the detection target region 38 as much as possible. The light emitting unit 68 may include a lens (not illustrated) at an intermediate position of the light guide path 78a so that the detection target region 38 is sufficiently irradiated with the first to fifth measurement light ml1 to ml5.

As in the light emitting unit 68, the light receiving unit 70 is disposed at a position facing the detection target region 38. The light receiving unit 70 has one or more (one in the present embodiment) light receiving elements 80, and a light guide portion 82 that is configured by the photometric block 54 and guides the transmitted light tl transmitted through the measurement tip 12 to the light receiving element 80. Hereinafter, first to fifth measurement light ml1 to ml5 transmitted through the measurement tip 12 are also referred to as first to fifth transmitted light tl1 to tl5, respectively.

The light receiving element 80 is connected to the detection circuit 72, and outputs a current corresponding to the amount of received light of the first to fifth transmitted light tl1 to tl5, to the detection circuit 72. As the light receiving element 80, for example, a photodiode (PD) can be adopted.

In addition, the light guide portion 82 of the light receiving unit 70 has a light guide path 82a that is configured by the photometric block 54 and guides the transmitted light tl to the light receiving element 80. In addition, the light guide portion 82 has a partition wall 83 between the mounting space 74 and the light guide path 82a. The partition wall 83 has an opening 83a through which the transmitted light tl of the measurement tip 12 is incident on the light guide path 82a. The partition wall 83 reduces scattering light which is scattered by the measurement tip 12 and directed to the light receiving element 80.

The detection circuit 72 controls power supply so that the first to fifth light emitting elements 76a to 76e of the light emitting unit 68 emit the light at a proper timing. In addition, the detection circuit 72 converts a detection current output from the light receiving element 80 into a voltage signal (detection value), and transmits the voltage signal to the control unit 88 of the measurement device 14.

Furthermore, in addition to the detection unit 64, the measurement device 14 internally has a power supply unit 84, a speaker 86, and the control unit 88. The power supply unit 84 has a dry cell or a battery, and supplies power to each electronic component of the measurement device 14. The speaker 86 outputs a proper voice or electronic sound under the control of the control unit 88.

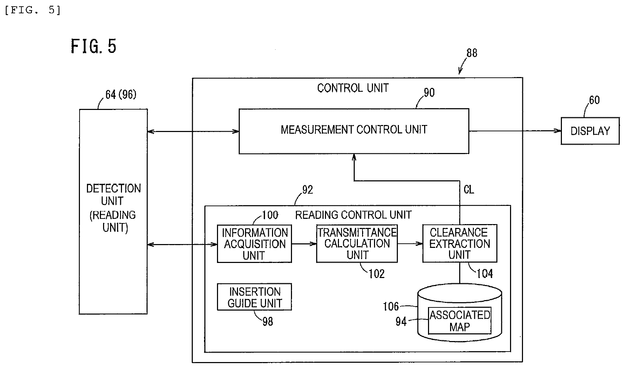

The control unit 88 is a computer having a processor, a memory, and an input/output interface. As the measurement device 14 is activated, the processor executes a program stored in the memory. In this manner, the control unit 88 internally builds a functional block for measuring the blood glucose level. Specifically, as illustrated in Fig. 5, a measurement control unit 90 and a reading control unit 92 are formed. In addition, after the measurement device 14 is activated, calibration is automatically performed before the measurement. In this manner, it is possible to reduce errors caused by measurement conditions. A result obtained through calibration before the measurement is stored in a reading control storage unit 106 (to be described later), and is used when the blood glucose level is calculated.

The measurement control unit 90 performs measurement control for measuring the blood glucose level by issuing a drive command to the detection circuit 72 at a proper timing. In the measurement control, when the blood is collected by the measurement tip 12 after the measurement tip 12 is mounted, the measurement control unit 90 sequentially emits the first to fifth measurement light ml1 to ml5 from the first to fifth light emitting elements 76a to 76e. In this manner, each of the first to fifth measurement light ml1 to ml5 transmits through the measurement tip 12, and the light receiving element 80 receives the first to fifth transmitted light tl1 to tl5 with a time lag. Thereafter, the light receiving element 80 outputs each detection value to the detection circuit 72. The measurement control unit 90 performs a calculation process of the blood glucose level, based on the detection value of the first to fifth transmitted light tl1 to tl5.

In the calculation process, for example, an absorbance is calculated using the detection value based on the first transmitted light t11, and a predetermined calculation process is further performed to calculate a provisional blood glucose level from the absorbance. The absorbance is calculated from the detection value of the transmitted light tl received by the light receiving element 80. First, the detection value of transmitted light tl'1 before the measurement tip 12 is mounted is recorded in the control unit 88 before the measurement starts. Thereafter, the measurement tip 12 is mounted. After a blood droplet is marked, the detection value of the transmitted light tl is measured, thereby calculating a logarithm of a numerical value obtained by dividing the detection value of the transmitted light tl by the detection value of the transmitted light tl'1 before the measurement tip 12 is mounted. Each absorbance is calculated for the detection target region 38 and the printing layer 18. However, both of these are 0 before the blood droplet is marked. When the absorbance is calculated, the measurement control unit 90 performs correction by using the information on the spatial clearance CL read in advance by the measurement device 14. Reading the spatial clearance CL will be described later.

In addition, during the calculation process, the absorbance or the provisional blood glucose level in the calculation process is corrected by using the detection value based on the second to fifth transmitted light tl2 to tl5, and a predetermined calculation process or map. In this manner, the blood glucose level as the measurement result is finally calculated. In the measurement control, the measurement control unit 90 may guide the blood droplet marking by using the display 60 or the speaker 86 after the measurement tip 12 is mounted. In addition, after the measurement control, the measurement result may be automatically stored in association with the date and time. Furthermore, in an activated state of the measurement device 14, the measurement control unit 90 reads the measurement result stored in the memory under the operation of the user, and performs a process of displaying the measurement result on the display 60.

On the other hand, when the user inserts the measurement tip 12 into the mounting space 74, the reading control unit 92 uses the detection unit 64 (reading unit 96) to perform tip information acquisition control for reading the information on the printing layer 18. Hereinafter, the tip information acquisition control will be described in detail.

As described above, the printing layer 18 is disposed in the tip body 16 so as to hold the information relating to the spatial clearance CL of the measurement tip 12. The spatial clearance CL is measured after the tip body 16 is manufactured, and the printing layer 18 is printed on (applied to) the tip body 16 by a printer (not illustrated) in a color based on the measured spatial clearance CL. Without being limited to the printing, the printing layer 18 can adopt a method of attaching a preprinted seal portion. In this manner, it is possible to reduce the disposal of the tip body 16 due to a printing failure. It is preferable to adopt a method of directly printing on the tip body 16, since there is no seal detachment danger or erroneous seal application, and the cost is low.

The spatial clearance CL is basically designed to be 40 µm. However, there is a possibility that the spatial clearance CL may be changed in a range of approximately 10 µm (for example, approximately 36 µm to 44 µm) due to a manufacturing error. The change in the spatial clearance CL is a major factor in degrading the measurement accuracy of the blood glucose level. Therefore, the blood glucose meter 10 is configured as follows. A plurality of colors to be printed as the printing layer 18 are associated with the spatial clearance CL, and the information relating to the spatial clearance CL is provided for the measurement device 14 by the measurement tip 12.

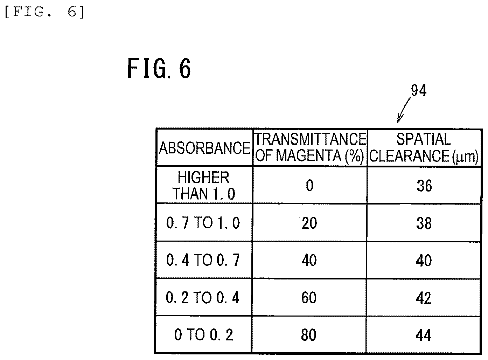

Specifically, as indicated by an associated map 94 illustrated in Fig. 6, magenta (C=0, M=100, Y=0, and K=0 in a CMYK value) is used as a color configuring the printing layer 18. A transmittance of the magenta is classified into five levels of 0%, 20%, 40%, 60%, and 80%. Then, the transmittance 0% is associated with the spatial clearance CL=36 µm, the transmittance 20% is associated with the spatial clearance CL=38 µm, the transmittance 40% is associated with the spatial clearance CL=40 µm, the transmittance 60% is associated with the spatial clearance CL=42 µm, and the transmittance 80% is associated with the spatial clearance CL=44 µm. The measurement device 14 has the associated map 94 in advance. Here, the "transmittance" is a set value for printing density, and is a ratio (%) of the transmitted light tl having a complementary color in the printing layer 18 to the transmitted light tl'1 before the printing. As the transmittance is lower, the application amount of ink applied by the printing increases.

The detection unit 64 of the measurement device 14 performs optical detection on the printing layer 18 when the measurement tip 12 is inserted, and also serves as the reading unit 96 that reads the transmittance of the magenta. That is, as illustrated in Fig. 6, the absorbance of the printing layer 18 detected by the detection unit 64 is lowered in a stepwise manner in accordance with the transmittance of magenta (0%, 20%, 40%, 60%, and 80%). For example, whereas the absorbance of magenta in a case of the transmittance 0% is 1.0, the absorbance of magenta in a case of the transmittance 80% is approximately 0.1.

In other words, the detection unit 64 can detect a change in the transmittance of the printing layer 18, and the measurement tip 12 can carry identifiable information in accordance with the change in the transmittance. The absorbance detected by the detection unit 64 slightly fluctuates each time of the detection. Accordingly, the measurement device 14 may set an allowable range for identifying the information (transmittance of the magenta). For example, as indicated by the associated map 94 in Fig. 6, a range is designated as follows. In a case where the absorbance is higher than 1.0, the transmittance of the magenta is 0%. In a range where the absorbance is 0.7 to 1.0, the transmittance of the magenta is 20%. In a range where the absorbance is 0.4% to 0.7%, the transmittance of the magenta is 40%. In a range where the absorbance is 0.2% to 0.4, the transmittance of the magenta is 60%. In a range where the absorbance is 0.0% to 0.2%, the transmittance of the magenta is 80%. Alternatively, the allowable range may be set to a predetermined plus or minus value (for example, ±0.04) with respect to a stepwise reference value (1.1, 0.85, 0.55, 0.3, and 0.1) of the absorbance.

Referring back to Fig. 5, as a functional unit for performing the tip information acquisition control, the reading control unit 92 of the measurement device 14 has an insertion guide unit 98, an information acquisition unit 100, a transmittance calculation unit 102, and a clearance extraction unit 104. In addition, the reading control unit 92 internally has the reading control storage unit 106 (memory storage region) for storing the above-described associated map 94 and storing various types of information when the tip information acquisition control is performed.

The insertion guide unit 98 guides the insertion of the measurement tip 12 by using the display 60 or the speaker 86 when the blood glucose level is measured.

The information acquisition unit 100 controls the driving of the detection unit 64 when the measurement tip 12 is inserted, and reads the information on the printing layer 18 of the measurement tip 12. For example, when the calibration before the measurement of the blood glucose level is completed, the information acquisition unit 100 controls the detection circuit 72 so that the fourth light emitting element 76d repeatedly emits the fourth measurement light ml4 at a predetermined reading interval (for example, 9 ms). The magenta includes the peak wavelength of approximately 550 nm. Accordingly, the magenta is likely to absorb the fourth measurement light ml4 having the peak wavelength of 540 nm. The light receiving unit 70 detects the fourth measurement light ml4 or the fourth transmitted light t14 transmitted through the measurement tip 12 by the emission of the fourth measurement light ml4, and outputs the detection result to the control unit 88 via the detection circuit 72. The information acquisition unit 100 outputs the detection result to the transmittance calculation unit 102. In addition to the magenta, an ink color used for the printing layer 18 may be selected from black, yellow, cyan, or a combination thereof. As the measurement wavelength, a wavelength suitable for reading the printing layer 18 and the detection target region 38 may be appropriately selected.

The transmittance calculation unit 102 calculates the absorbance each time the detection result is received from the information acquisition unit 100. The absorbance can be calculated in the same manner as the calculation of the absorbance in the measurement control. Then, the transmittance calculation unit 102 monitors a change in the absorbance. In a case where the absorbance is changed as much as a predetermined value (for example, 0.15) or greater compared to the absorbance before the measurement tip 12 reaches the detection unit 64 and the change is continued for a predetermined time (for example, 45 ms) or longer, the information of the absorbance is output to the clearance extraction unit 104.

The clearance extraction unit 104 reads the information of the associated map 94 (refer to Fig. 6) stored in the reading control storage unit 106 during the tip information acquisition control. Then, when the absorbance is received from the transmittance calculation unit 102, the spatial clearance CL corresponding to the absorbance is extracted (specified) with reference to the associated map 94. Furthermore, the clearance extraction unit 104 outputs the spatial clearance CL to the measurement control unit 90, and stores the spatial clearance CL in the reading control storage unit 106.

Therefore, during the measurement control, the measurement control unit 90 can accurately calculate the blood glucose level by using the spatial clearance CL received from the clearance extraction unit 104 (reading control unit 92).

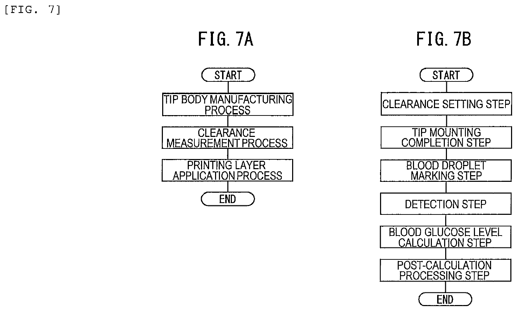

Next, manufacturing of the measurement tip 12 having the above-described configuration, and an operation of the blood glucose meter 10 when the measurement tip 12 is used will be described with reference to flowcharts illustrated in Figs.7A and 7B.

In the manufacturing of the measurement tip 12, as illustrated in Fig.7A, a tip body manufacturing process, a clearance measurement process, and a printing layer application process are sequentially performed.

In the tip body manufacturing process, the upper member 26 and the lower member 24 are respectively injection-molded by an injection molding machine (not illustrated). The upper member 26 and the lower member 24 may be manufactured using not only the injection molding but also a film. In addition, after the injection molding, in the lower member 24, the reagent 40 is applied to the detection target region 38. Then, the tip body 16 is manufactured so that the upper member 26 and the lower member 24 are stacked on each other and both members are fixed to each other by fixing means.

In the clearance measurement process, the spatial clearance CL of the tip body 16 manufactured as described above is measured. For example, the detection target region 38 of the tip body 16 is irradiated with the light having a predetermined wavelength by using an optical film thickness measurement device or a laser film thickness measurement device. A state of the reflected wave (phase change) is detected. In this manner, the spatial clearance CL (interval between the reagent 40 and the upper member 26) can be measured. In addition, without being limited to measuring the tip body 16 after the tip body 16 is manufactured, in the clearance measurement process, a value calculated from lots or manufacturing conditions of a member to be used may be substituted therewith, or may be used in combination. For example, the lower member 24, the upper member 26 and a spacer used for fixing may be adopted. This method contributes to further improved accuracy.

In the printing layer application process, an ink jet or laser printer is used. The magenta having the transmittance corresponding to the spatial clearance CL measured in the clearance measurement process is printed on the upper member 26 side of the tip body 16. In this manner, each of the measurement tips 12 has the printing layer 18 having a color relating to the spatial clearance CL.

As illustrated in Fig. 7B, in measuring the blood glucose level, the measurement tip 12 manufactured by the above-described manufacturing method and the measurement device 14 are used. First, an interval setting step of acquiring the spatial clearance CL of the measurement tip 12 is performed.

In the interval setting step, after the calibration is performed before the measurement of the measurement device 14, the insertion guide unit 98 of the control unit 88 guides the user to mount the measurement tip 12 on the measurement device 14. Based on the guidance, the user inserts the measurement tip 12 into the mounting space 74 of the photometric block 54.

As illustrated in Fig. 8A, when the measurement tip 12 is inserted, the information acquisition unit 100 drives the light emitting unit 68 (fourth light emitting element 76d) to repeatedly emit the fourth measurement light ml4. The light receiving unit 70 receives the fourth measurement light ml4 or the fourth transmitted light tl4 transmitted through the measurement tip 12. The information acquisition unit 100 receives the detection result from the detection circuit 72, and provides the detection result for the transmittance calculation unit 102.

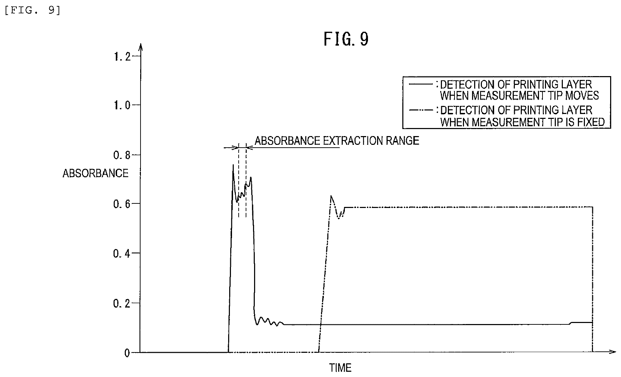

The transmittance calculation unit 102 calculates a low absorbance before the printing layer 18 passes. However, the transmittance calculation unit 102 calculates a high absorbance when the printing layer 18 passes through the detection unit 64 as illustrated in Fig. 8B. That is, as illustrated in Fig. 9, the measurement device 14 detects approximately 0 as the absorbance before the measurement tip 12 passes. However, the measurement device 14 detects sharply raised absorbance when the inserted printing layer 18 passes.

Instead of detecting a moment when the absorbance is sharply raised, the reading control unit 92 may determine that the printing layer 18 is read, based on a continuous state where the absorbance rises for a predetermined period of time. In addition, it is preferable that the reading control unit 92 calculates an average value from a plurality of values in a state where the absorbance is raised. Furthermore, when the average value is calculated, it is preferable to extract an intermediate period during the rise of the absorbance so that overshooting at an initial detection stage is not included. According to this configuration, when the detection unit 64 reads the printing layer 18, the measurement tip 12 is inserted and moved. It means a condition under which the detection value is likely to include noise. In contrast, the reading control unit 92 calculates the average value. Accordingly, the influence of the noise can be reduced to some extent. In addition, for the detection value used in calculating the average value, the intermediate period is extracted. Accordingly, the influence of the noise can be further reduced, and the accuracy in calculating the transmittance can be improved.

As illustrated in Fig. 5, when the transmittance calculation unit 102 detects a great change in the absorbance, the transmittance calculation unit 102 calculates the transmittance from the absorbance, and outputs the transmittance to the clearance extraction unit 104. The clearance extraction unit 104 extracts the spatial clearance CL corresponding to the transmittance, based on the transmittance calculated by the transmittance calculation unit 102 and the read associated map 94. Then, the clearance extraction unit 104 stores the extracted spatial clearance CL in the reading control storage unit 106, and outputs the extracted spatial clearance CL to the measurement control unit 90. In this manner, the interval setting step is completed. A threshold may be set for the change in the absorbance detected by the transmittance calculation unit 102. In a case where the change in the absorbance exceeding the threshold is detected, it may be recognized that the measurement tip 12 is inserted, and the measurement control unit 90 may be activated. In this manner, even if the measurement device 14 is in an activated state, and in a case where the change in the absorbance is not detected for a long time, the measurement control unit 90 may be brought into a deactivated state to minimize power consumption. The activation and the deactivation are switched by detecting the printing layer 18. Accordingly, compared to a case where the switching is performed by detecting other portions, the measurement device 14 can be activated with accuracy and less power.

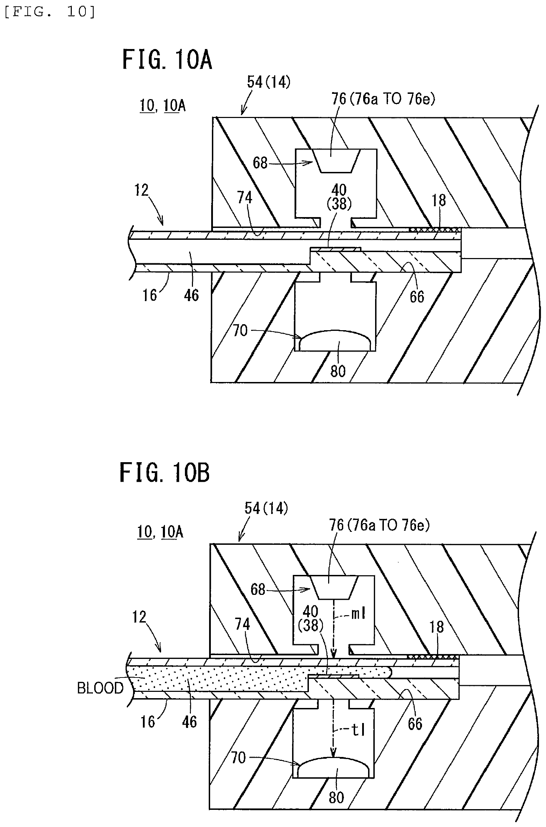

The insertion guide unit 98 further inserts the measurement tip 12 even after the printing layer 18 passes through the detection unit 64. The user inserts the measurement tip 12 to the inner portion of the measurement device 14. In this manner, as illustrated in Fig. 10A, the detection target region 38 is caused to face the detection unit 64. When the measurement tip 12 reaches the inner portion (tip mounting completion step in Fig. 7B), the tip information acquisition control is completed. Subsequently, the process proceeds to the measurement control.

Referring back to Fig. 7B, in the measurement control, the mounted measurement tip 12 is calibrated before the measurement, and the user is prompted so that the blood droplet is marked on the measurement tip 12 (blood droplet marking step). In this manner, the user marks the blood droplet on the cutout 42 of the measurement tip 12. As illustrated in Fig. 10B, when the blood flows through the blood flow path 46 of the measurement tip 12 in the proximal direction and reaches the detection target region 38, the blood is colored after reacting with the reagent 40.

The measurement control unit 90 drives the light emitting unit 68 and the light receiving unit 70 to optically detect the detection target region 38 (detection step). As described above, in the present embodiment, the light emitting unit 68 sequentially emits the first to fifth measurement light ml1 to ml5, and the light receiving unit 70 receives the first to fifth transmitted light tl1 to tl5.

The measurement control unit 90 receives the detection result of the first to fifth transmitted light tl1 to tl5, and calculates the blood glucose level (blood glucose level calculation step). At this time, the measurement control unit 90 calculates the absorbance from the measurement result, uses the spatial clearance CL provided from the reading control unit 92, and uses a parameter of the spatial clearance CL to calculate the blood glucose level.

In this manner, the measurement device 14 can accurately obtain the blood glucose level. Then, the measurement control unit 90 stores the blood glucose level in the memory, and displays the blood glucose level on the display 60 to notify the user of the blood glucose level (post-calculation processing step).

As described above, the blood glucose meter 10 (component measurement system 10) according to the present embodiment achieves the following advantageous effects.

The measurement tip 12 can easily provide the information relating to the spatial clearance CL by using the printing layer 18. On the other hand, the measurement device 14 can easily acquire the information relating to the spatial clearance CL by using the reading unit 96. In this manner, the blood glucose meter 10 can accurately obtain the blood glucose level, based on each spatial clearance CL of the measurement tip 12. As a result, a health care worker can more satisfactorily perform treatment corresponding to the blood glucose level of a patient. In particular, the printing layer 18 is printed after the tip body 16 is manufactured. Accordingly, the manufacturing error is less likely to occur. Therefore, the printing layer 18 can be easily provided at low cost, thereby contributing to mass production of the measurement tip 12.

In addition, the blood glucose meter 10 associates the information relating to the spatial clearance CL with the transmittance of the printing layer 18. Therefore, the measurement device 14 can accurately recognize the spatial clearance CL of the measurement tip 12 by calculating the transmittance of the printing layer 18.

Furthermore, the blood glucose meter 10 is configured to detect the transmitted light tl of the measurement tip 12 by using the detection unit 64. Accordingly, the blood glucose level can be satisfactorily calculated by using the blood collected in the measurement tip 12, based on the absorbance of the measurement light ml.

Then, when the measurement tip 12 is inserted and moved, the blood glucose meter 10 detects the transmitted light tl of the printing layer 18. In this manner, before the blood is collected by the measurement tip 12, the information relating to the spatial clearance CL can be easily obtained. In addition, a time can be shortened, compared to a case where the insertion is stopped once, when the printing layer 18 is read. Owing to the reduced procedures, an erroneous operation is less likely to occur. Moreover, the detection unit 64 also serves as the reading unit 96. Accordingly, the measurement device 14 is allowed to have a simplified configuration, and the manufacturing cost can be reduced. Without being limited to a top surface of the measurement tip 12, the printing layer 18 can also be disposed on an intermediate layer. In this case, the printing layer 18 can be prevented from being dirty or moisture absorption, and can be accurately detected even after the printing layer 18 is stored for a long period.

### [Second Embodiment]

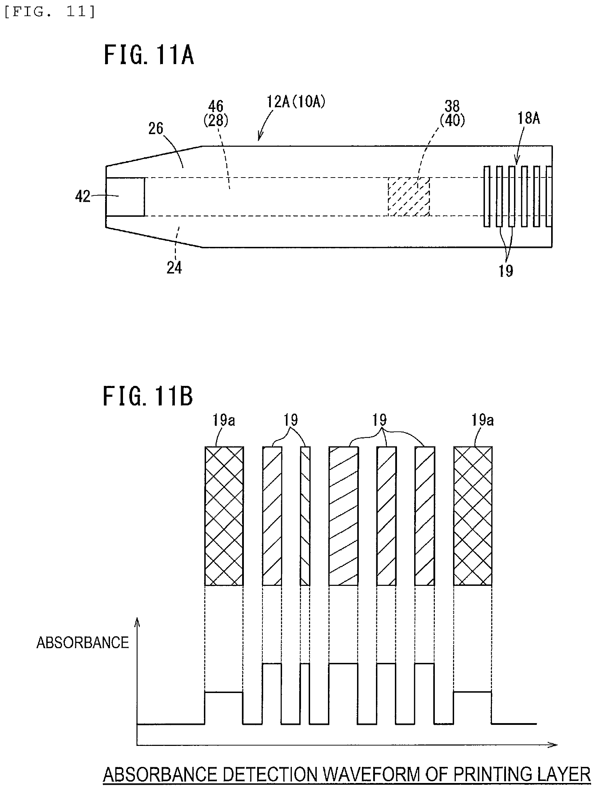

Next, a component measurement system 10A (blood glucose meter 10A) according to a second embodiment will be described with reference to Figs.11A and 11B. In the following description, the same reference numerals will be assigned to elements having the same configuration or the same function as those according to the above-described embodiment, and detailed description thereof will be omitted.

The blood glucose meter 10A is different from the blood glucose meter 10 according to the first embodiment in the following configuration. In the blood glucose meter 10A, a printing layer 18A of a measurement tip 12A has a pattern of a shape change capable of identifying the information relating to the spatial clearance CL, and the measurement device 14 can read the pattern to obtain the spatial clearance CL. The measurement device 14 has the same structure (the detection unit 64 and the control unit 88) as the measurement device 14 according to the first embodiment. The control unit 88 is configured to analyze the pattern of the shape change in the printing layer 18A (refer to Figs. 10A and 10B).

Specifically, in the printing layer 18A according to the present embodiment, a plurality of rectangular configuration elements 19 are aligned, based on the spatial clearance CL of the measurement tip 12A. In this manner, a pattern (a so-called barcode shape) is formed. For example, each configuration element 19 is formed in a predetermined color (magenta in the present embodiment) having the transmittance of 0%. Therefore, as the measurement tip 12A is inserted, the detection unit 64 of the measurement device 14 detects a portion where the configuration element 19 exists and a portion where the configuration element 19 does not exist, and outputs the detection result to the control unit 88 via the detection circuit 72. Here, an example will be described in which the configuration element 19 has a single color and the same transmittance, and the pattern shape is changed. However, the configuration element 19 may have the same pattern shape and the transmittance may be changed. Alternatively, a plurality of colors may be combined with each other. The amount of information can be increased by combining a plurality of the color schemes, the transmittances, and the patterns which are used for printing. Accordingly, it is possible to simultaneously input other correction factors in addition to the spatial clearance CL. In order to easily read the information of the printing layer 18A, a color scheme having a contrast may be provided between the configuration elements 19 adjacent to each other.

That is, the reading control unit 92 of the control unit 88 acquires approximately two types of signal values. In this case, the transmittance calculation unit 102 calculates two types of the absorbance (low absorbance and high absorbance), based on the signal values, and converts the absorbance into binary numbers of 0 and 1. The clearance extraction unit 104 has an associated map (not illustrated) in which a plurality of the spatial clearances CL and the binary numbers are associated with each other, and can extract the spatial clearance CL, based on the binary numbers received from the transmittance calculation unit 102.

Reading the pattern of the measurement tip 12A by the measurement device 14 is affected by an insertion amount or an insertion speed of the measurement tip 12A. Therefore, as illustrated in Fig. 11B, in addition to the pattern of the configuration element 19, it is preferable that a check bit 19a indicating a reading start position, a reading completion position, or reading intermediate position is formed on the printing layer 18A of the measurement tip 12A. In Fig. 11B, the check bit 19a is installed in front of and behind the configuration element 19 by widening its width in the longitudinal direction of the measurement tip 12A. However, a plurality of the color schemes, the transmittances, and the patterns may be combined with each other so that a difference from the configuration element 19 is clear. Alternatively, it is also possible to express the difference by repeating a constant signal. A start portion may be omitted, and all of these may be appropriately selected and set.

For example, the measurement device 14 causes the detection unit 64 to read each of two check bits 19a, causes the control unit 88 to calculate the insertion speed by measuring a time interval between the two check bits 19a, and sets a threshold. In this manner, the measurement device 14 determines whether the insertion speed of the measurement tip 12A is normal or abnormal. Then, in a case of determining that the insertion speed falls within the threshold and the insertion speed is normal, the measurement device 14 acquires the above-described spatial clearance CL. On the other hand, in a case where it is determined that the insertion speed is abnormal, the measurement device 14 instructs the measurement tip 12A to be inserted again. In this manner, the measurement device 14 can satisfactorily obtain the spatial clearance CL.

As described above, the blood glucose meter 10A according to the second embodiment can also achieve the same advantageous effect as that according to the above-described blood glucose meter 10. In particular, in the blood glucose meter 10A, the information relating to the spatial clearance CL is associated with the pattern of the shape change in the printing layer 18A. According to this configuration, it is also possible to accurately recognize the spatial clearance CL of the measurement tip 12A by detecting the pattern of the shape change in the printing layer 18A in the measurement device 14. Moreover, the pattern of the shape change in the printing layer 18A can hold a plurality of types of information at the same time. For example, in addition to the information on the spatial clearance CL of the measurement tip 12A, information such as a manufacturing date, a lot, and a correction value can be provided for the measurement device 14.

### [Third Embodiment]

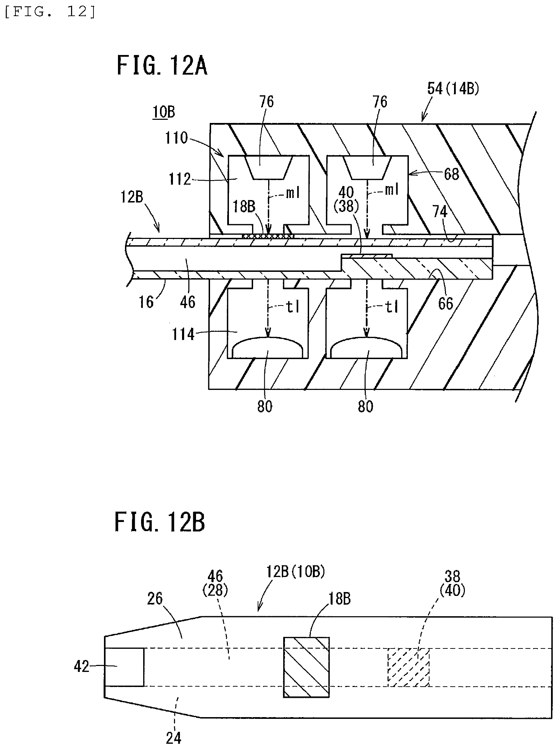

A blood glucose meter 10B according to a third embodiment is different from the above-described blood glucose meters 10 and 10A in the following points. As illustrated in Figs. 12A and 12B, one of the plurality of light emitting elements 76 of the detection unit 64 is disposed on the distal side of other elements so that a reading unit 110 detects a printing layer 18B of a measurement tip 12B. In addition, the blood glucose meter 10B is configured to detect the printing layer 18B in a state where the measurement tip 12B is mounted on a measurement device 14B (at a completely mounted position).

The printing layer 18B of the measurement tip 12B is disposed on the upper surface of the insertion range 20 on the distal side (outer side in the insertion direction) of the detection target region 38. For example, the printing layer 18B is located at a substantially intermediate position in the longitudinal direction of the measurement tip 12B, and is disposed to face the reading unit 110 at the completely mounted position.

In addition, the printing layer 18B according to the present embodiment is configured to have different colors in accordance with the spatial clearance CL of the measurement tip 12B to be printed. For example, the colors are classified into yellow, magenta, cyan, an intermediate color between yellow and magenta, an intermediate color between magenta and cyan, and an intermediate color between yellow and cyan, and are set to have a predetermined transmittance. Then, in the blood glucose meter 10B, the spatial clearance CL is associated with each of the classified colors.

The reading unit 110 of the measurement device 14B has a printing layer light emitting unit 112 that emits the measurement light ml having a predetermined peak wavelength (or white light), and a printing layer light receiving unit 114 that receives the transmitted light t1 transmitted through the measurement tip 12B. In this case, the printing layer light receiving unit 114 can output different detection values to the control unit 88, based on different colors of the printing layer 18B.

The printing layer light emitting unit 112 and the light emitting unit 68 are respectively driven. In this manner, based on the different detection values, the reading control unit 92 of the measurement device 14B can identify the information as in the blood glucose meter 10 according to the first embodiment. In other words, the reading control unit 92 can extract the spatial clearance CL of the measurement tip 12B. In addition, the measurement device 14B drives the reading unit 110 when the blood glucose level is measured. In this manner, the measurement device 14B can optically detect the blood which is not colored. For example, the detection value can be used for correction when the blood glucose level is calculated.

As described above, the blood glucose meter 10B according to the third embodiment can also achieve the same advantageous effects as those according to the above-described blood glucose meter 10. In particular, in the blood glucose meter 10B, the information relating to the spatial clearance CL is associated with the color of the printing layer 18B. According to this configuration, it is also possible to accurately recognize the spatial clearance CL of the measurement tip 12B by detecting the color of the printing layer 18B in the measurement device 14B.

In addition, the blood glucose meter 10B detects the transmitted light tl of the printing layer 18B in a mounted state of the measurement tip 12B. That is, as illustrated by a two-dot chain line in a graph in Fig. 9, when the printing layer 18B is detected in a state where the measurement tip 12B is fixed, the absorbance is stabilized, and a detection target portion can be extracted. In this manner, it is possible to more reliably obtain the information relating to the spatial clearance CL. Moreover, in the blood glucose meter 10B, some of the elements of the detection unit 64 also serve as the reading unit 110. Therefore, a configuration of the measurement device 14B is simplified, and the manufacturing cost can be reduced.

### [Fourth Embodiment]

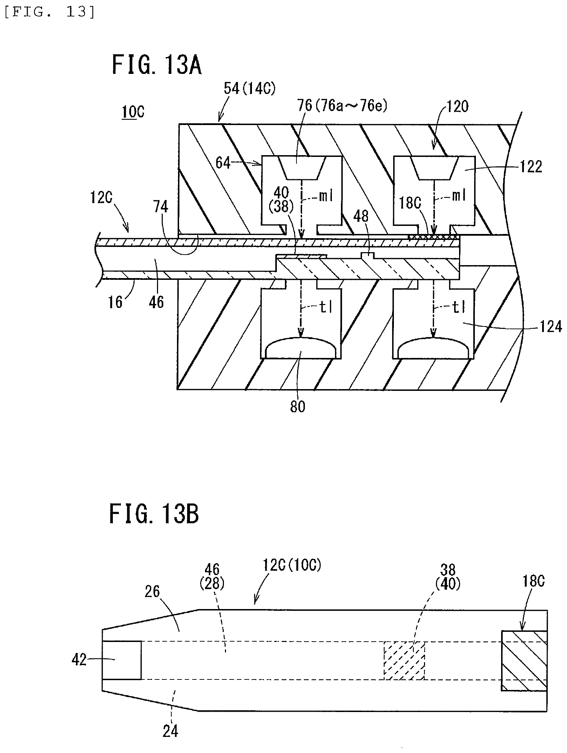

A blood glucose meter 10C according to a fourth embodiment is different from the above-described blood glucose meters 10, 10A, and 10B in the following points. As illustrated in Figs. 13A and 13B, in the mounting space 74, a proximal side of the detection unit 64 has a printing layer reading unit 120 that exclusively detects the information on a printing layer 18C of a measurement tip 12C. As in the blood glucose meter 10B according to the third embodiment, the blood glucose meter 10C detects the printing layer 18C in a state where the measurement tip 12C is mounted on a measurement device 14C (at a completely mounted position).

As in the measurement tip 12 according to the first embodiment, the printing layer 18C of the measurement tip 12C is disposed on an upper surface of a proximal portion (inner side of the detection target region 38 in the insertion direction) of the measurement tip 12C, and is disposed to face the printing layer reading unit 120 at the completely mounted position. In addition, the blood flow path 46 of the measurement tip 12C has a blocking wall 48 for blocking a flow of the blood between the detection target region 38 and the printing layer 18C. Therefore, when blood is collected in the measurement tip 12C, the blood does not flow into a lower side of the printing layer 18C.

In addition, the printing layer 18C is configured to have different colors and different transmittances in accordance with the spatial clearance CL. For example, the colors are classified into yellow, magenta, and cyan, and each color is classified into the transmittance 0% and the transmittance 50%. Then, in the blood glucose meter 10C, the spatial clearance CL is associated with each combination of each classified color and transmittance.

On the other hand, the printing layer reading unit 120 of the measurement device 14C has a printing layer light emitting unit 122 and a printing layer light receiving unit 124, and outputs different detection values to the control unit 88, based on the different colors and the different transmittances of the printing layer 18C of the measurement tip 12C. Therefore, as in the blood glucose meter 10 according to the first embodiment, the control unit 88 can satisfactorily extract the spatial clearance CL of the measurement tip 12C.

As described above, the blood glucose meter 10C according to the fourth embodiment can also achieve the same advantageous effects as those according to the above-described blood glucose meter 10. In particular, as in the blood glucose meter 10B according to the third embodiment, the blood glucose meter 10C detects the transmitted light tl of the printing layer 18C in a mounted state of the measurement tip 12C. In this manner, the blood glucose meter 10C can more reliably obtain the information relating to the spatial clearance CL. In addition, the blood glucose meter 10C transmits the information relating to the spatial clearance CL by using both the color and the transmittance of the printing layer 18C. Accordingly, a lot of information can be transmitted. For example, it is possible to improve resolution of the spatial clearance CL.

### Non-claimed example

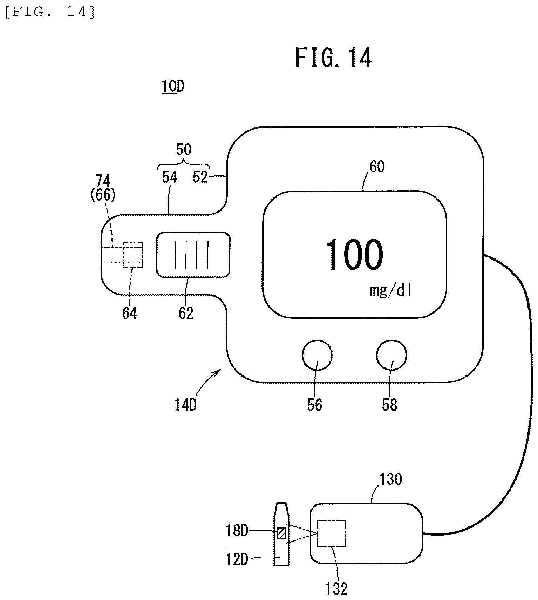

A blood glucose meter 10D according to a non-claimed example is different from the above-described blood glucose meters 10 and 10A to 10C in the following point. As illustrated in Fig. 14, a measurement device 14D has a reader 130 for exclusively reading a printing layer 18D of a measurement tip 12D before the mounting, separately from the detection unit 64. In addition, according to the present example, the reader 130 is configured to include a camera 132 that can image a color and a shape of the printing layer 18D.

The camera 132 adopts those which have a known image element such as a CMOS, and can image and acquire image information such as the color and the shape of the printing layer 18D. The control unit 88 of the measurement device 14D performs an analyzing process on the image information captured by the camera 132, and extracts the color and the shape of the printing layer 18D. Then, the control unit 88 compares the color and the shape of the printing layer 18D with the stored information (color, shape, and measurement space information) to satisfactorily obtain the spatial clearance CL. In this manner, the blood glucose level can be satisfactorily calculated.

The blood glucose meter 10D according to the non-claimed example can also achieve the same advantageous effects as those according to the above-described blood glucose meter 10. In addition, the blood glucose meter 10D uses the camera 132 as a configuration for identifying the printing layer 18D. Accordingly, the blood glucose meter 10D can transmit more information from the measurement tip 12D to the measurement device 14D. For example, a plurality of types of information (measurement result, measurement date and time, manufacturing date, lot, and correction information) relating to the measurement target and/or the measurement condition in addition to the spatial clearance CL of the measurement tip 12D can be stored in association with each other. In this manner, the measurement tip 12D can calculate and manage the blood glucose level by associating the plurality of types of information with each other.

Without being limited to the above-described embodiments, the present invention may be modified in various ways and is defined by the appended claims. Any configuration of one embodiment can be selected from the above-described first to fifth embodiments, and can be applied to the other embodiment.

For example, without being limited to a transmission type optical system, the detection unit 64 of the blood glucose meters 10, 10A to 10D may be configured to include a reflective type optical system in which the light emitting unit 68 and the light receiving unit 70 are disposed on the same side to receive the measurement light (reflected light) reflected on the measurement tips 12 and 12A to 12D. In accordance with this configuration, the reading unit 96 may also be configured to be the reflective type.

Furthermore, in the above-described embodiments, a simplified self-monitoring blood glucose meter (SMBG meter) among the blood glucose meters 10 and 10A to 10D for measuring the blood glucose level has been described as an example. However, without being limited to the above-described embodiments, various modifications can be made within the scope of the invention, which is defined by the appended claims. For example, the component measurement systems 10 and 10A to 10D can be applied to a device for very accurately measuring concentration, such as a hospital dedicated glucose analyzer (POCT device) or a large-scale test apparatus installed in a medical facility or a research facility. The component measurement systems 10 and 10A to 10D can be applied to a simplified measurement device that not only calculates the concentration, but also determines whether or not a detection threshold has been exceeded when a small amount is detected. In addition to the blood, a sample liquid as a measurement target at a medical site includes a solution of a sample obtained from a living body such as urine (ketone body), interstitial fluid, and saliva. The sample liquid may be an undiluted solution, or an experimental product subjected to chemical treatment. Alternatively, the component measurement systems 10 and 10A to 10D can be applied to an apparatus for measuring the component of waste water or industrial samples. In brief, the component measurement systems 10 and 10A to 10D can be applied to (combined with) various devices that fetch and measure the liquid.

In addition, the information held by the printing layers 18 and 18A to 18D is not limited to the information relating to the measurement of the spatial clearance CL, and may be information relating to the liquid collected by the measurement tips 12 and 12A to 12D and/or the measurement conditions. As described above, examples of the liquid include blood, urine, interstitial fluid, saliva, waste water, and industrial samples. The printing layers 18 and 18A to 18D hold types of the liquid to be measured as information. For example, in a case of measuring a ketone body and urine sugar which are contained in urine, the information relating to the types of the measurement tips 12 and 12A to 12D is printed on the printing layers 18 and 18A to 18D. The information is read by the measurement devices 14 and 14B to 14D. The measurement devices 14 and 14B to 14D calculate the measurement value in accordance with the type of the measurement tips 12 and 12A to 12D, and store the information relating to the type of the measurement tips 12 and 12A to 12D in association with the measurement result. In this manner, the same measurement devices 14 and 14B to 14D are used to accurately measure the plurality of components contained in the liquid. Accordingly, it is possible to effectively utilize the measurement results. For example, the measurement conditions include conditions of temperature, humidity, and light (color, intensity, and brightness) during the measurement (when the measurement tips 12 and 12A to 12D are used after being unpacked). As the information, the printing layers 18 and 18A to 18D hold a range of these measurement conditions. A temperature-responsive ink, a humidity-responsive ink, or an ultraviolet coloring ink may be used for the printing. For example, in a case where the temperature-responsive ink is printed on the measurement tips 12 and 12A to 12D, a coloring change in the temperature-responsive ink corresponding to the temperature of the measurement tips 12 and 12A to 12D is read by the measurement devices 14 and 14B to 14D. The measurement devices 14 and 14B to 14D measure a temperature condition of a portion close to a measurement site by detecting the coloring change in the temperature-responsive ink, and uses the temperature condition for the correction. In this manner, it is possible to more accurately calculate the measurement value. In addition, for example, in a case where the humidity-responsive ink and the ultraviolet coloring ink are printed on the measurement tips 12 and 12A to 12D, when the measurement tips 12 and 12A to 12D are unpacked and used, the measurement devices 14 and 14B to 14D read changes in moisture absorption and absorbance which result from unpacking of the measurement devices, as the coloring changes in the printing layers 18 and 18A to 18D. The measurement devices 14 and 14B to 14D can determine whether or not to perform the measurement by not only performing correction for the coloring change, but also setting a threshold corresponding to the coloring change of the printing layers 18 and 18A to 18D. In this way, the printing layers 18 and 18A to 18D hold the range of the measurement condition. Accordingly, more accurate measurement can be performed, and more accurate measurement management can be performed.

In addition, in a case where the component of the above-described liquid is measured, without being limited to the devices that perform the optical measurement, the measurement devices 14 and 14B to 14D (component measurement systems 10 and 10A to 10D) may adopt various devices. For example, the applicable device includes an electrical measurement device (device that measures a current or a voltage through the liquid), a device that detects properties (products, morphology, temperature, viscosity (coagulation or liquefaction) which are changed due to a reaction between the liquid and the reagent, and a device that extracts a specific component from the liquid. When the measurement is performed, the measurement devices 14 and 14B to 14D can correct the measurement value by using the information relating to the liquid and/or the measurement condition. For example, when the liquid is measured, the information relating to the measurement condition (temperature, humidity, light) is detected. In a case where the detection value falls out of the information relating to the measurement condition, a correction amount is calculated, based on the detection value and the information relating to the measurement condition. In this manner, the measurement value can be appropriately corrected.

In addition, the measurement devices 14 and 14B to 14D may be configured to provide the information to the user by not only using the information relating to the liquid and/or the measurement condition for calculation in the measurement, but also storing the information in association with the calculated measurement value. Alternatively, the information relating to the liquid and/or the measurement condition may be managed as data in another information management system. As the information management system, a PC, a mobile phone, an electronic medical record system, or a server may be used. The information management system can be more conveniently used if the information can be transmitted and received among the measurement devices 14 and 14B to 14D by means of wireless communication. In this way, the information relating to the liquid and/or the measurement condition and the measurement value are managed in association with each other. Accordingly, it is possible to recognize the measurement value including a measurement status when the measurement value is confirmed or statistical processing is performed.

## Claims

1. A component measurement system (10) comprising:
a measurement tip (12) capable of collecting a liquid; and
a measurement device (14) measuring a component of the liquid by emitting measurement light to the measurement tip (12), the measurement tip being mounted on the measurement device (14),
wherein the measurement tip (12) has
a tip body (16) internally having a detection space into which the liquid is fetched, and a detection target region (38) irradiated with the measurement light in the detection space, and
a printing layer (18) disposed on a surface of the tip body (16), and indicating information relating to a spatial clearance (CL) which is an interval of the detection space along a traveling direction of the measurement light in the detection target region (38), and
wherein the measurement device (14) has
a reading unit (96) that reads the information relating to the spatial clearance (CL) from the printing layer (18), and
a control unit (88) that calculates the component of the liquid by using the spatial clearance (CL) read by the reading unit (96)
**characterized in that**
the measurement device (14) includes a detection unit (64) that emits the measurement light to detect transmitted light transmitted through the measurement tip (12),
the measurement device (14) has a mounting space (74) into which the measurement tip (12) is inserted,
the measurement tip (12) has the printing layer (18) located on an inner side from the detection target region (38) in an insertion direction of the measurement tip (12) in a state mounted in the measurement device (14), and
the detection unit (64) also functions as the reading unit (96), and is configured to detect transmitted light of the printing layer (18), when the measurement tip (12) is inserted and moved.

2. A component measurement system (10) comprising:
a measurement tip (12) capable of collecting a liquid; and
a measurement device (14) measuring a component of the liquid by emitting measurement light to the measurement tip (12), the measurement tip being mounted on the measurement device (14),
wherein the measurement tip (12) has
a tip body (16) internally having a detection space into which the liquid is fetched, and a detection target region (38) irradiated with the measurement light in the detection space, and
a printing layer (18) disposed on a surface of the tip body (16), and indicating information relating to a spatial clearance (CL) which is an interval of the detection space along a traveling direction of the measurement light in the detection target region (38), and
wherein the measurement device (14) has
a reading unit (96) that reads the information relating to the spatial clearance (CL) from the printing layer (18), and
a control unit (88) that calculates the component of the liquid by using the spatial clearance (CL) read by the reading unit (96),
**characterized in that**
the measurement device (14) includes a detection unit (64) that emits the measurement light to detect transmitted light transmitted through the measurement tip (12),
the measurement device (14) has a mounting space (74) into which the measurement tip (12) is inserted,
the measurement tip (12) has the printing layer (18) located on an outer side from the detection target region (38) in an insertion direction of the measurement tip (12) in a state mounted in the measurement device (14), and
the detection unit (64) also functions as the reading unit (96), and is configured to detect transmitted light of the printing layer (18), in a state mounted in the measurement device (14).

3. The component measurement system (10) according to Claim 1 or 2,
wherein the control unit (88) specifies the spatial clearance (CL), based on a transmittance of the printing layer (18).

4. The component measurement system (10) according to Claim 1 or 2,
wherein the control unit (88) specifies the spatial clearance (CL), based on a color of the printing layer (18).

5. The component measurement system (10) according to Claim 1 or 2,
wherein the control unit (88) specifies the spatial clearance (CL), based on a pattern of a shape change in the printing layer (18).

6. The component measurement system (10) according to Claim 1 or 2,
wherein the printing layer (18) holds information relating to the liquid and/or a measurement condition by being printed.

7. The component measurement system (10) according to Claim 6,
wherein the information relating to the liquid and/or the measurement condition is stored by being associated with the measurement value.

## Patentansprüche

1. Komponentenmesssystem (10) umfassend:
eine Messspitze (12), die in der Lage ist, eine Flüssigkeit zu sammeln; und
eine Messvorrichtung (14), die eine Komponente der Flüssigkeit durch Aussenden von Messlicht an die Messspitze (12) misst, wobei die Messspitze an der Messvorrichtung (14) angebracht ist,
wobei die Messspitze (12) aufweist einen Spitzenkörper (16), der im Inneren einen Erfassungsraum aufweist, in welchen die Flüssigkeit hereingeholt wird, und einen Erfassungszielbereich (38), der mit dem Messlicht in dem Erfassungsraum bestrahlt wird, und
eine Druckschicht (18), die auf einer Oberfläche des Spitzenkörpers (16) angeordnet ist und Informationen anzeigt, die sich auf einen räumlichen Abstand (CL) beziehen, der ein Intervall des Erfassungsraums entlang einer Ausbreitungsrichtung des Messlichts in dem Erfassungszielbereich (38) ist, und
wobei die Messvorrichtung (14) aufweist eine Leseeinheit (96), welche die Information bezüglich des räumlichen Abstands (CL) von der Druckschicht (18) liest, und
eine Steuerungseinheit (88), die den Anteil der Flüssigkeit unter Verwendung des von der Leseeinheit (96) gelesenen räumlichen Abstands (CL) berechnet **dadurch gekennzeichnet, dass**
die Messvorrichtung (14) eine Erfassungseinheit (64) umfasst, die das Messlicht aussendet, um durchgelassenes Licht zu erfassen, das durch die Messspitze (12) durchgelassen wird,
die Messvorrichtung (14) einen Aufnahmeraum (74) aufweist, in welchen die Messspitze (12) eingesetzt wird, die Messspitze (12) die Druckschicht (18) aufweist, die auf einer Innenseite von dem Erfassungszielbereich (38) in einer Einführungsrichtung der Messspitze (12) in einem Zustand angeordnet ist, in dem sie in der Messvorrichtung (14) angebracht ist, und
die Erfassungseinheit (64) auch als die Leseeinheit (96) fungiert und so konfiguriert ist, dass sie durchgelassenes Licht der Druckschicht (18) erfasst, wenn die Messspitze (12) eingeführt und bewegt wird.

2. Komponentenmesssystem (10) umfassend:
eine Messspitze (12), die in der Lage ist, eine Flüssigkeit zu sammeln; und
eine Messvorrichtung (14), die eine Komponente der Flüssigkeit durch Aussenden von Messlicht an die Messspitze (12) misst, wobei die Messspitze an der Messvorrichtung (14) angebracht ist,
wobei die Messspitze (12) aufweist
einen Spitzenkörper (16), der im Inneren einen Erfassungsraum aufweist, in welchen die Flüssigkeit hereingeholt wird, und einen Erfassungszielbereich (38), der mit dem Messlicht in dem Erfassungsraum bestrahlt wird, und
eine Druckschicht (18), die auf einer Oberfläche des Spitzenkörpers (16) angeordnet ist und Informationen anzeigt, die sich auf einen räumlichen Abstand (CL) beziehen, der ein Intervall des Erfassungsraums entlang einer Ausbreitungsrichtung des Messlichts in dem Erfassungszielbereich (38) ist, und
wobei die Messvorrichtung (14) aufweist
eine Leseeinheit (96), welche die Information bezüglich des räumlichen Abstands (CL) von der Druckschicht (18) liest, und
eine Steuerungseinheit (88), die den Anteil der Flüssigkeit unter Verwendung des von der Leseeinheit (96) gelesenen räumlichen Abstands (CL) berechnet,
**dadurch gekennzeichnet, dass**
die Messvorrichtung (14) eine Erfassungseinheit (64) umfasst, die das Messlicht aussendet, um durchgelassenes Licht zu erfassen, das durch die Messspitze (12) durchgelassen wird,
die Messvorrichtung (14) einen Aufnahmeraum (74) aufweist, in welchen die Messspitze (12) eingesetzt wird, die Messspitze (12) die Druckschicht (18) aufweist, die auf einer Innenseite von dem Erfassungszielbereich (38) in einer Einführungsrichtung der Messspitze (12) in einem Zustand angeordnet ist, in dem sie in der Messvorrichtung (14) angebracht ist, und
die Erfassungseinheit (64) auch als die Leseeinheit (96) funktioniert und so konfiguriert ist, dass sie durchgelassenes Licht der Druckschicht (18) erfasst, in einem Zustand, in dem sie in der Messvorrichtung (14) angebracht ist.

3. Komponentenmesssystem (10) nach Anspruch 1 oder 2, wobei die Steuerungseinheit (88) den räumlichen Abstand (CL) auf der Grundlage eines Transmissionsgrades der Druckschicht (18) spezifiziert.

4. Komponentenmesssystem (10) nach Anspruch 1 oder 2, wobei die Steuerungseinheit (88) den räumlichen Abstand (CL) auf der Grundlage einer Farbe der Druckschicht (18) spezifiziert.

5. Komponentenmesssystem (10) nach Anspruch 1 oder 2, wobei die Steuerungseinheit (88) den räumlichen Abstand (CL) auf der Grundlage eines Musters einer Formänderung in der Druckschicht (18) spezifiziert.

6. Komponentenmesssystem (10) nach Anspruch 1 oder 2, wobei die Druckschicht (18) Informationen bezüglich der Flüssigkeit und/oder einer Messbedingung enthält, indem sie gedruckt wird.

7. Komponentenmesssystem (10) nach Anspruch 6,
wobei die Information bezüglich der Flüssigkeit und/oder der Messbedingung gespeichert wird, indem sie dem Messwert zugeordnet wird.

## Revendications

1. Système de mesure de composant (10) comprenant :
une pointe de mesure (12) capable de collecter un liquide ; et
un dispositif de mesure (14) mesurant un composant du liquide par l'émission d'une lumière de mesure vers la pointe de mesure (12), la pointe de mesure étant montée sur le dispositif de mesure (14),
dans lequel la pointe de mesure (12) comporte
un corps de pointe (16) comportant, à l'intérieur de celui-ci, un espace de détection dans lequel le liquide est récupéré, et une région cible de détection (38) irradiée avec la lumière de mesure dans l'espace de détection, et
une couche d'impression (18) disposée sur une surface du corps de pointe (16), et indiquant des informations relatives à un dégagement spatial (CL) qui est un intervalle de l'espace de détection le long d'une direction de déplacement de la lumière de mesure dans la région cible de détection (38), et
dans lequel le dispositif de mesure (14) comporte
une unité de lecture (96) qui lit les informations relatives au dégagement spatial (CL) depuis la couche d'impression (18), et
une unité de commande (88) qui calcule le composant du liquide par l'utilisation du dégagement spatial (CL) lu par l'unité de lecture (96)
**caractérisé en ce que**
le dispositif de mesure (14) inclut une unité de détection (64) qui émet la lumière de mesure pour détecter une lumière transmise qui est transmise à travers la pointe de mesure (12),
le dispositif de mesure (14) comporte un espace de montage (74) dans lequel la pointe de mesure (12) est insérée,
la pointe de mesure (12) comporte la couche d'impression (18) située sur un côté intérieur depuis la région cible de détection (38) dans une direction d'insertion de la pointe de mesure (12) dans un état monté dans le dispositif de mesure (14), et
l'unité de détection (64) fonctionne également en tant que l'unité de lecture (96), et est configurée pour détecter une lumière transmise de la couche d'impression (18), lorsque la pointe de mesure (12) est insérée et déplacée.

2. Système de mesure de composant (10) comprenant :
une pointe de mesure (12) capable de collecter un liquide ; et
un dispositif de mesure (14) mesurant un composant du liquide par l'émission d'une lumière de mesure vers la pointe de mesure (12), la pointe de mesure étant montée sur le dispositif de mesure (14),
dans lequel la pointe de mesure (12) comporte
un corps de pointe (16) comportant, à l'intérieur de celui-ci, un espace de détection dans lequel le liquide est récupéré, et une région cible de détection (38) irradiée avec la lumière de mesure dans l'espace de détection, et
une couche d'impression (18) disposée sur une surface du corps de pointe (16), et indiquant des informations relatives à un dégagement spatial (CL) qui est un intervalle de l'espace de détection le long d'une direction de déplacement de la lumière de mesure dans la région cible de détection (38), et
dans lequel le dispositif de mesure (14) comporte
une unité de lecture (96) qui lit les informations relatives au dégagement spatial (CL) depuis la couche d'impression (18), et
une unité de commande (88) qui calcule le composant du liquide par l'utilisation du dégagement spatial (CL) lu par l'unité de lecture (96),
**caractérisé en ce que**
le dispositif de mesure (14) inclut une unité de détection (64) qui émet la lumière de mesure pour détecter une lumière transmise qui est transmise à travers la pointe de mesure (12),
le dispositif de mesure (14) comporte un espace de montage (74) dans lequel la pointe de mesure (12) est insérée,
la pointe de mesure (12) comporte la couche d'impression (18) située sur un côté extérieur depuis la région cible de détection (38) dans une direction d'insertion de la pointe de mesure (12) dans un état monté dans le dispositif de mesure (14), et
l'unité de détection (64) fonctionne également en tant que l'unité de lecture (96), et est configurée pour détecter une lumière transmise de la couche d'impression (18), dans un état monté dans le dispositif de mesure (14) .

3. Système de mesure de composant (10) selon la revendication 1 ou 2,
dans lequel l'unité de commande (88) spécifie le dégagement spatial (CL), sur la base d'une transmittance de la couche d'impression (18).

4. Système de mesure de composant (10) selon la revendication 1 ou 2,
dans lequel l'unité de commande (88) spécifie le dégagement spatial (CL), sur la base d'une couleur de la couche d'impression (18).

5. Système de mesure de composant (10) selon la revendication 1 ou 2,
dans lequel l'unité de commande (88) spécifie le dégagement spatial (CL), sur la base d'un motif d'un changement de forme dans la couche d'impression (18).

6. Système de mesure de composant (10) selon la revendication 1 ou 2,
dans lequel la couche d'impression (18) contient des informations relatives au liquide et/ou à une condition de mesure en étant imprimée.

7. Système de mesure de composant (10) selon la revendication 6,
dans lequel les informations relatives au liquide et/ou à la condition de mesure sont stockées en étant associées à la valeur de mesure.
